# EUROPEAN PATENT SPECIFICATION

(11) **EP 2 995 952 B1**
(45) Date of publication and mention of the grant of the patent: **12.06.2019**
(21) Application number: 15181977.8
(22) Date of filing: 21.08.2015
(51) Int. Cl.: G01N 33/53, G01N 33/543, G01N 33/553, G01N 33/58, G01N 33/74

(54) **REAGENT KIT, MEASUREMENT KIT, AND METHOD OF MEASURING TEST SUBSTANCE**
REAGENZKIT, MESSKIT UND VERFAHREN ZUR MESSUNG EINER TESTSUBSTANZ
KIT DE RÉACTIF, KIT DE MESURE ET PROCÉDÉ DE MESURE D'UNE SUBSTANCE D'ESSAI

(30) Priority: 09.09.2014 JP 2014183064
(43) Date of publication of application: 16.03.2016
(73) Proprietor: Fujifilm Corporation, Minato-ku Tokyo 106-8620 (JP)
(72) Inventor: Nakamura, Kazuhiro, Kanagawa, 258-8538 (JP); Kasagi, Noriyuki, Kanagawa, 258-8538 (JP); Chiku, Hiroyuki, Kanagawa, 258-8538 (JP); Nakamura, Kentaro, Kanagawa, 258-8538 (JP)
(74) Representative: Klunker IP Patentanwälte PartG mbB

(56) References cited:
- EP-A1- 2 562 185
- EP-A2- 1 321 770
- WO-A1-2007/138964
- WO-A2-02/27316
- US-A- 5 219 763
- US-A1- 2006 257 926

## Description

### BACKGROUND OF THE INVENTION

### 1. Field of the Invention

The present invention relates to a reagent kit which is used for measuring a test substance in a biological sample, a measurement kit which is used for measuring the test substance in the biological sample, and a method of measuring the test substance. The test substance is adrenocorticotropic hormone (ACTH).

### 2. Description of the Related Art

A fluorescence detection method is widely used as an easy measurement method with high sensitivity for quantitatively determining a test substance, such as protein, an enzyme, or an inorganic compound, which is contained in a biological sample. The fluorescence detection method is a method of confirming the presence of the test substance by detecting fluorescence which is emitted when irradiating a test sample, which is considered to contain a test substance emitting fluorescence by being excited by light of a specific wavelength, with excitation light of the above-described specific wavelength. In a case where the test substance is not a fluorescent body, it is possible to confirm the presence of the test substance by detecting fluorescence which is emitted when the excitation light is emitted similarly to the above after bringing a substance which is obtained by labeling a substance which specifically binds to the test substance with a fluorescent pigment, into contact with the test sample.

In the above-described fluorescence detection method, a method of utilizing an effect of electric field enhancement using plasmon resonance, in order to improve the sensitivity of detection is known. In this method, a sensor chip which is obtained by providing a metal film in a predetermined region on a transparent support body is prepared in order to generate the plasmon resonance. Then, excitation light is made to be incident on the interface between the support body and the metal film at an angle greater than or equal to a total reflection angle from a surface side opposite to the surface on which the metal film is formed in the support body. Surface plasmons are generated on the metal film by emitting the excitation light, fluorescence is enhanced by an electric field enhancement action due to the generation of the surface plasmons, and the signal/noise ratio (S/N ratio) is improved. In the fluorescence detection method using surface plasmon excitation (hereinafter, referred to as "SPF method"), an enhancement degree 10 times that in a fluorescence detection method using vertical illumination excitation is obtained.

JP2568910B discloses an immunoassay method of an antigen in a liquid sample which includes a stage in which a complex is formed between the antigen and at least two antibodies, one of which is a first antibody that binds to an insoluble support body and the other of which is a second unbound labeling antibody, and a part of the above-described labeling antibody binds to the insoluble support body through the above-described complex; and a stage in which an additional antibody which is selected from the group consisting of an unlabeled unbound first antibody, unlabeled unbound second antibody, and a mixture thereof is introduced such that there is a sufficient amount for causing a pseudo first order reaction to obtain a standard curve which is substantially in a linear form.

JP5492158B and EP 2 562 185 A1 disclose a method of measuring thyroid stimulating hormone (TSH) using SPF using a monoclonal antibody which is produced by a specific hybridoma and can bind to human TSH and dog TSH.

US 5,219,763 A discloses a reagent kit comprising fluorescent beads coated with poly-L-Lysine and beads coated with an anti-aflatoxin monoclonal antibody.

WO 0 227 316 A2 discloses an assay and the reagents therefor for the detection of an analyte (e.g. TSH, PSA). The reagents used comprise a buffer with a polycation such as poly-ornithine, poly-lysine, poly-histidine, antibody coated paramagnetic microparticles, and a particle consisting of an antibody acridinium labeled conjugate.

WO 2007 138 964 A1 disclose a colored latex particle to which a binding substance is coupled and a buffer comprising arginine. Also disclosed is the use of these reagents in an immunoassay for the detection of analytes, such as chorionic gonadotropin, a C-reactive protein and a myoglobin.

EP 1 321 770 A2 discloses an immunoassay for the detection of an analyte such as gentamicin. The assay involves the use of fluorescent latex particles conjugated with an antibody and a buffer comprising triethanolamine.

US 2006 257926 A1 discloses reagents as well as their use in an immunoassay for the detection of an analyte, said reagents comprising latex particles conjugated with the rabbit anti-human lipoprotein antibodies and a buffer comprising arginine.

### SUMMARY OF THE INVENTION

As described above, JP2568910B discloses an immunoassay method of an antigen using a sandwich immunity reaction using two antibodies, but the measurement sensitivity is not sufficient. In addition, JP5492158B relates to a method of measuring TSH using a monoclonal antibody which is produced by a deposited specific hybridoma. However, JP5492158B does not provide a highly sensitive measurement method for an arbitrary test substance.

An object of the present invention is to provide a reagent kit which is used for measuring a test substance (ACTH) in a biological sample and can improve the measurement sensitivity of the test substance; a measurement kit which contains the above-described reagent kit; and a method of measuring the test substance which can improve the measurement sensitivity of the test substance.

The present inventors have conducted extensive studies in order to solve the above-described problems, and as a result, they have found that it is possible to suppress nonspecific reaction between a substrate and first particles which are modified by a first binding substance having specific binding properties with respect to a test substance (ACTH) and have a label, and to improve the measurement sensitivity of the test substance, by performing a reaction in the presence of second particles which are modified by a second binding substance which does not have specific binding properties with respect to the test substance, the second particles not having a label, and in the presence of a compound which has at least one amino group having a positive charge when reacting the test substance with the above-described first particles having a label. The present invention has been completed based on the knowledge. That is, according to the present invention, the following invention is provided.
(1) A reagent kit which is used for measuring a test substance in a biological sample, including: first particles which are modified by a first binding substance having specific binding properties with respect to the test substance and have a label; second particles which are modified by a second binding substance which does not have specific binding properties with respect to the test substance, the second particles not having a label, and a compound which has at least one amino group having a positive charge, and has a isoelectric point greater than 7, wherein the first binding substance is anti-ACTH antibody, wherein the second binding substance is anti-CRP antibody, wherein the test substance is adrenocorticotropic hormone, and wherein the mass ratio of the second particles to the first particles is 1 to 6.
(2) The reagent kit according to (1) in which the compound is an amino acid.
(3) The reagent kit according to (2) in which the amino acid is at least one compound which is selected from arginine, histidine, lysine, and ornithine.
(4) The reagent kit according to any one of (1) to (3) in which the particle size of the first particles is 200 nm to 300 nm.
(5) The reagent kit according to any one of (1) to (4) in which the label contains a fluorescent pigment.
(6) The reagent kit according to any one of (1) to (5) in which the particle size of the second particles is 100 nm to 200 nm.
(7) The reagent kit according to any one of (1) to (6) in which the amount of the compound, which has at least one amino group having a positive charge and has an isoelectric point greater than 7, used is 100 mmol/L to 400 mmol/L in terms of concentration in a reaction liquid.
(8) A measurement kit which is used for measuring a test substance in a biological sample, including: the reagent kit according to any one of (1) to (7); and a substrate on which a first metal film, to which a third binding substance having specific binding properties with respect to the test substance, or a substance having binding properties with respect to the first binding substance, is immobilized is formed.
(9) The measurement kit according to (8) in which a second metal film, to which a fourth binding substance which does not have binding properties with respect to the test substance and has binding properties with respect to the first binding substance, is immobilized is further formed on the substrate.
(10) A method of measuring a test substance, including: a step of bringing a solution, which contains a test substance, first particles which are modified by a first binding substance having specific binding properties with respect to the test substance and have a label, second particles which are modified by a second binding substance which does not have specific binding properties with respect to the test substance, the second particles not having a label, and a compound which has at least one amino group having a positive charge, and has an isoelectric point greater than 7, into contact with a first metal film to which a third binding substance having specific binding properties with respect to the test substance, or a substance having binding properties with respect to the first binding substance is immobilized; and a step of detecting a signal in accordance with the label from the first metal film, wherein the first binding substance is anti-ACTH antibody, wherein the second binding substance is anti-CRP antibody, wherein the test substance is adrenocorticotropic hormone, and wherein the mass ratio of the second particles to the first particles is (1) to (6).
(11) The measurement method according to (10), further including: a step of bringing the solution into contact with a second metal film to which a fourth binding substance which does not have binding properties with respect to the test substance and has binding properties with respect to the first binding substance is immobilized; a step of detecting a signal in accordance with the label from the second metal film; and a step of correcting the signal detected from the first metal film using the signal detected from the second metal film.

According to the reagent kit, measurement kit, and the measurement method of the present invention, it is possible to improve the measurement sensitivity in measuring a test substance (ACTH) in a biological sample.

### DESCRIPTION OF THE PREFERRED EMBODIMENTS

Hereinafter, the present invention will be described in more detail.

### [Reagent Kit]

A reagent kit of the present invention is a kit for use in measuring a test substance (ACTH)in a biological sample and contains first particles which are modified by a first binding substance having specific binding properties with respect to the test substance and have a label and a compound which has at least one amino group having a positive charge. As described above, the reagent kit of the present invention contains the first particles and the compound which has at least one amino group having a positive charge. However, the first particles and the above-described compound may be contained in the kit as a mixture, or may be contained separately in the kit. Further, the kit contains second particles which are modified by a second binding substance which does not have specific binding properties with respect to the test substance, the second particles not having a label.

### (Biological Sample)

The biological sample is not particularly limited as long as there is a possibility that the sample may contain a test substance, and examples thereof include biological samples, in particular, body fluids (for example, blood, serum, plasma, cerebrospinal fluids, tears, sweat, urine, pus, nasal mucus, or expectoration) from animals (particularly a human), excretions (for example, feces), internal organs, tissues, mucous membranes, and skin.

### (Test Substance)

The test substance is adrenocorticotropic hormone (ACTH),.

Adrenocorticotropic hormone (ACTH) is a peptide hormone (molecular weight of 4,500) which is formed of 39 amino acids. ACTH is secreted from the pituitary gland, and the adrenal gland is stimulated to secrete cortisol. In diagnosis of the most common Cushing syndromes among metabolic diseases, ACTH is an important measurement item together with cortisol. Pituitary gland-originated pituitary dependent hyperadrenocorticism (PDH) and adrenal gland-originated adrenal tumor (AT) cause Cushing syndrome, and it is possible to diagnose that there is a high likelihood of PDH if the value of ACTH is high in a specimen with a high value of cortisol after an ACTH stimulation test, and there is a high likelihood of AT if the value of the ACTH is low in a specimen with a high value of cortisol after an ACTH stimulation test. Since ACTH does not form a steric structure, it readily decomposes into protease contained in blood. Therefore, it is necessary to treat blood which has been promptly adjusted in ethylenediaminetetraacetic acid plasma (EDTA) while cooling the specimen after collecting blood.

### (Compound Which Has at least One Amino Group Having Positive Charge)

The reagent kit of the present invention is a kit for use in measuring a test substance (ACTH) in a biological sample, and allows measurement of (preferably allows quantitative determination of) the test substance using an immunoreaction. In the measurement method according to the present invention, it is estimated that a third binding substance having specific binding properties with respect to the test substance (ACTH) or a first binding substance having binding properties with respect to the test substance binds to the test substance (ACTH) through an electrostatic interaction. In a case where latex particles which are negatively charged on the surface are selected as the first particles, there is a problem in that non-specific binding through an electrostatic interaction between the latex particles and an antibody on a gold film occurs, and therefore, it is impossible to obtain sufficient correlation between the concentration of the test substance and a detection signal and to obtain a calibration curve having sufficient sensitivity. In order to solve such a phenomenon, using a blocking agent with respect to the non-specific reaction may be considered, and in the present invention, it has been found that it is possible to solve the above-described problem using a compound which has at least one amino group having a positive charge, and has an isoelectric point greater than 7..

Specifically, the compound which has at least one amino group having a positive charge is preferably a compound which has an amino acid having an amino group in a side chain or has a structure similar to the above-described amino acid. The number of the amino groups having a positive charge is not particularly limited as long as being greater than or equal to 1, but is preferably 1 to 5 and more preferably 1 to 3. Whether the amino group in the compound has a positive charge can be determined from an isoelectric point of the compound. If the isoelectric point is greater than 7, it can be regarded that the amino acid has a positive charge in a biological sample in the vicinity of pH 7.

As the measurement method of the isoelectric point, it is possible to measure the isoelectric point using a usual surface potentiometer or a zeta potentiometer. Specifically, it is possible to employ the pH when the pH becomes 0 as the isoelectric point by preparing an aqueous solution of a compound to be measured and measuring the zeta potential of the aqueous solution using a commercially available surface potentiometer while changing the pH. Specific numerical values are arginine 10.76, lysine 9.74, histidine 7.59, and glycine 5.97.

Examples of an amino acid which has an amino group in a side chain and constitutes protein include arginine, histidine, and lysine. The isoelectric point of these amino acids is greater than 7, and therefore, the fact that the amino acids have a positive charge in a biological sample in the vicinity of pH 7 is supported. In addition, it is also possible to use a compound which has a structure similar to ornithine such as guanidine hydrochloride other than the amino acids, and arginine and lysine are particularly preferable.

The amount of the compound which has at least one amino group having a positive charge used is preferably 50 mmol/L to 1000 mmol/L, more preferably 100 mmol/L to 400 mmol/L, and still more preferably 150 mmol/L to 275 mmol/L, in terms of concentration in a reaction liquid.

### (First Binding Substance)

The first binding substance used in the present invention is anti-ACTH antibody, which is a substance having specific binding properties with respect to the test substance. Generally, in a case where the binding substance is an antibody, as an antibody having specific binding properties with respect to a test substance, it is possible to use, for example, antiserum which is prepared from serum of an animal which has been immunized with the test substance; an immunoglobulin fraction which has been purified from the antiserum; and a monoclonal antibody obtained through cell fusion using a spleen cell of an animal which has been immunized with the test substance; or a piece thereof [for example, F(ab')2, Fab, Fab', or Fv]. The preparation of these antibodies can be performed through a usual method. Furthermore, the antibodies may be antibodies which are modified as in a case of a chimeric antibody. Alternately, commercially available antibodies or antibodies which have been prepared through a well-known method from animal serum or a culture supernatant can also be used.

The antibodies can be used regardless of the species or the subclass of the animal. Specific examples of the antibodies which can be used in the present invention include antibodies, such as mouse IgG, mouse IgM, rat IgG, rat IgM, hamster IgG, hamster IgM, rabbit IgG, rabbit IgM, goat IgG, goat IgM, sheep IgG, sheep IgM, cattle IgG, cattle IgM, and chicken IgY, which are derived from organisms, such as mice, rats, hamsters, goats, rabbits, sheep, cattle, and chickens, in which an immunoreaction can occur. Either of polyclonal antibodies or monoclonal antibodies can be used.

Particularly, since the test substance is ACTH, an anti-ACTH monoclonal antibody is preferably used as a first binding substance having specific binding properties with respect to the test substance (ACTH) through an electrostatic interaction. A sandwich system is preferably selected in consideration of the molecular weight of ACTH. In this case, it is necessary to coat a gold film with an antibody which becomes a pair. It is possible to use anti-ACTH monoclonal antibodies with the gold film and first particles on both of the gold film and the first particles.

### (First Particles)

The first particles used in the present invention are particles which are modified by the above-described first binding substance and have a label. The first particles are preferably dried particles, but are not limited. It is possible to use, for example, polymer particles such as polystyrene beads and glass particles such as glass beads as the first particles which can be usually used for immunoassay. Specific examples of the quality of the material of the first particles include a polymer using a monomer such as styrene, methacrylic acid, glycidyl (meth)acrylate, butadiene, vinyl chloride, vinyl acetate acrylate, methyl methacrylate, ethyl methacrylate, phenyl methacrylate, or butyl methacrylate, or a synthetic polymer such as a copolymer using two or more monomers. Latex which is obtained by uniformly suspending monomers is preferable. In addition, other examples thereof include an organic polymer powder, an inorganic substance powder, a microorganism, a piece of a blood corpuscle or a cell membrane, or liposome.

In a case of using latex particles, specific examples of the quality of the material of the latex include polystyrene, a styrene-acrylic acid copolymer, a styrene-methacrylic acid copolymer, a styrene-glycidyl (meth)acrylate copolymer, a styrene-styrene sulfonate copolymer, a methacrylic acid polymer, an acrylic acid polymer, an acrylonitrile-butadiene-styrene copolymer, a vinyl chloride-acrylic acid ester copolymer, and a polyvinyl acetate acrylate. As the latex, a copolymer containing at least styrene as a monomer is preferable and a copolymer of styrene and acrylic acid or methacrylic acid is particularly preferable. The method of producing the latex is not particularly limited, and it is possible to produce the latex through an arbitrary polymerization method. However, it is difficult to immobilize an antibody if there is a surfactant during antibody labeling. Therefore, when producing the latex, emulsifier-free emulsion polymerization, that is, emulsion polymerization without using an emulsifier such as a surfactant is preferable.

The first particles have a label. The label preferably emits fluorescence. When the latex itself which is obtained through polymerization is fluorescent, it is possible to use the latex as fluorescent latex particles. When the latex which is obtained through polymerization is non-fluorescent, it is possible to produce the fluorescent latex particles by adding a fluorescent substance (such as fluorescent pigment) to the latex. That is, the fluorescent latex particles can be produced by impregnating a fluorescent pigment into latex particles by adding the fluorescent pigment to a solution of the latex particles containing water and water soluble organic solvent and stirring the fluorescent pigment and the solution.

As the first particles having a label, it is possible to use a liposome, a microcapsule, or the like containing a fluorescent pigment as the fluorescent particles. The fluorescent color development is not particularly limited as long as ultraviolet light or the like is absorbed and causes excitement, and fluorescence is emitted during return to the ground state, and examples thereof include fluorescent color developments of yellowish green (excitation wavelength 505 nm / emission wavelength 515: the same applies hereinafter), blue (350 nm to 356 nm / 415 nm to 440 nm), red (535 nm to 580 nm / 575 nm to 605 nm), orange (540 nm / 560 nm), red orange (565 nm / 580 nm), crimson 625 nm / 645 nm), and dark red (660 nm / 680 nm). Fluorescent particles which emit these kinds of fluorescence can be obtained from, for example, Invitrogen Corporation, and are commercially available with a product name of FluoSpheres (registered trade name) from Invitrogen Corporation.

The particle size of the first particles having a label is defined as an average particle size. The average particle size of the first particles is not particularly limited. A preferred range of the average particle size differs depending on the quality of the material of the particles, the concentration range in which the test substance is quantitatively determined, a measurement instrument, and the like, and is preferably 200 nm to 300 nm, more preferably 220 to 290, and still more preferably 240 nm to 280 nm.

The average particle size of the particles used in the present invention can be measured by a commercially available particle size distribution meter. As the measurement method of the particle distribution, optical microscopy, confocal laser microscopy, electron microscopy, atomic force microscopy, a static light scattering method, a laser diffraction method, a dynamic light scattering method, a centrifugal sedimentation method, an electric pulse measurement method, a chromatography method, and an ultrasonic attenuation are known, and apparatuses corresponding to the principles are commercially available. Among the measurement methods, the particle distribution is preferably measured using a dynamic light scattering method. In the present invention, the average particle size is obtained as a median diameter (50% diameter, d50) which is measured under the conditions of a viscosity of 0.8872 CP (0.8872 mPA·s) and a refractive index of water of 1.330 at 25°C.

The first particles used in the present invention are modified by the above-described first binding substance. The method for binding the first binding substance to the first particles having a label is not particularly limited. For example, the method thereof is disclosed in JP2000-206115A or a protocol attached to FluoSpheres (registered trade name) polystyrene microspheres F8813 of Molecular Probes Inc. Any well-known method can be used in which a reagent is prepared for an immunoagglutination reaction. In addition, as a method of immobilizing a binding substance such as an antibody on particles, any method such as a method using physical adsorption or a method using chemical binding through covalent bonding can also be employed. As a blocking agent which covers the surfaces of particles which are not coated with a binding substance such as an antibody after immobilizing the binding substance to the particles, well-known substances, for example, bovine serum albumin (BSA), skim milk, casein, soy-derived components, fish-derived components, or polyethylene glycol, or commercially available blocking agents for immunoreaction containing these substances or substances having the same characteristics as these substances can be used. It is possible to perform pretreatment, such as partial modification through heat, acid/alkali or the like, on these blocking agents as necessary.

### (Second Binding Substance)

The reagent kit of the present invention further contains second particles which are modified by a second binding substance which does not have specific binding properties with respect to a test substance.

There is a test sample which becomes positive through a reaction even with a negative test sample which does not contain a test substance as well as with a test sample which becomes positive and contains a test substance, and solving the problem of this false positive is recognized as a task. The cause of such false positives is not clear, but it is considered that a non-specific reaction occurring due to presence of some factor contained in serum may be one cause of the false positives. In the present invention, such a problem is solved by additionally using the second particles which are modified by the second binding substance which does not have specific binding properties with respect to the test substance.

As the second binding substance, a substance is used which does not have specific binding properties with respect to the test substance (ACTH). It is preferable to use a substance that may bind to a substance causing the above-described false positive and to further use a compound that does not have affinity to the first binding substance. As the second binding substance, an antibody is used. Generally, in a case where the second binding substance is an antibody, it is possible to use an antiserum which is prepared from the serum of an animal which has been immunized with an antigen thereof; an immunoglobulin fraction which has been purified from the antiserum; and a monoclonal antibody obtained through cell fusion using a spleen cell of an animal which has been immunized with the test substance; or a piece thereof [for example, F(ab')2, Fab, Fab', or Fv]. The preparation of these antibodies can be performed through a usual method. Furthermore, the antibodies may be antibodies which are modified as in a case of a chimeric antibody. Alternatively, commercially available antibodies or antibodies which have been prepared through a well-known method from animal serum or a culture supernatant can also be used. In the present invention, an anti-CRP antibody as the second binding substance is used.

### (Second Particles)

The second particles do not have a label and are preferably dried particles, but are not particularly limited.

It is possible to use, for example, polymer particles such as polystyrene beads and glass particles such as glass beads as the second particles which can be usually used for immunoassay. Specific examples of the quality of the material of the second particles include a polymer using a monomer such as styrene, methacrylic acid, glycidyl (meth)acrylate, butadiene, vinyl chloride, vinyl acetate acrylate, methyl methacrylate, ethyl methacrylate, phenyl methacrylate, or butyl methacrylate, or a synthetic polymer such as a copolymer using two or more monomers. Latex which is obtained by uniformly suspending monomers is preferable. In addition, other examples thereof include an organic polymer powder, an inorganic substance powder, a microorganism, a piece of a blood corpuscle or a cell membrane, or a liposome.

In a case of using latex particles, specific examples of the quality of the material of the latex include polystyrene, a styrene-acrylic acid copolymer, a styrene-methacrylic acid copolymer, a styrene-glycidyl (meth)acrylate copolymer, a styrene-styrene sulfonate copolymer, a methacrylic acid polymer, an acrylic acid polymer, an acrylonitrile-butadiene-styrene copolymer, a vinyl chloride-acrylic acid ester copolymer, and polyvinyl acetate acrylate. As the latex, a copolymer containing at least styrene as a monomer is preferable and a copolymer of styrene and acrylic acid or methacrylic acid is particularly preferable. The method of producing the latex is not particularly limited, and it is possible to produce the latex through an arbitrary polymerization method. However, it is difficult to immobilize an antibody if there is a surfactant during antibody labeling. Therefore, when producing the latex, emulsifier-free emulsion polymerization, that is, emulsion polymerization without using an emulsifier such as a surfactant is preferable.

The particle size of the second particles which bind by the second binding substance is defined as an average particle size. The average particle size of the second particles differs depending on the quality of the material of the particles, the concentration range in which the test substance is quantitatively determined, a measurement instrument, and the like, and is preferably 100 nm to 200 nm, more preferably 120 to 180, and still more preferably 130 nm to 170 nm.

With respect to the use ratio of the second particles to the first particles, the mass ratio of the second particles to the first particles is 1 to 6 and more preferably 2 to 6.

### [Measurement Kit]

The measurement kit of the present invention contains the above-described reagent kit of the present invention; and a substrate on which a first metal film, to which a third binding substance having specific binding properties with respect to a test substance, or a substance having binding properties with respect to the first binding substance, is immobilized is formed. A second metal film, to which a fourth binding substance which does not have binding properties with respect to the test substance and has binding properties with respect to the first binding substance, is immobilized may be further formed on the above-described substrate.

### (Third Binding Substance)

The third binding substance is not particularly limited as long as the third binding substance has specific binding properties with respect to a test substance. Preferred examples thereof include an antigen, an antibody, or a complex thereof, and an antibody is preferable. In a case where the third binding substance is an antibody, as an antibody having specific binding properties with respect to a test substance, it is possible to use, for example, an antiserum which is prepared from the serum of an animal which has been immunized with the test substance; an immunoglobulin fraction which has been purified from the antiserum; and a monoclonal antibody obtained through cell fusion using a spleen cell of an animal which has been immunized with the test substance; or a piece thereof [for example, F(ab')2, Fab, Fab', or Fv]. The preparation of these antibodies can be performed through a usual method. Furthermore, the antibodies may be antibodies which are modified as in a case of a chimeric antibody. Alternately, commercially available antibodies or antibodies which have been prepared through a well-known method from animal serum or a culture supernatant can also be used.

The antibodies can be used regardless of the species or the subclass of the animal. Specific examples of the antibodies which can be used in the present invention include antibodies, such as mouse IgG, mouse IgM, rat IgG, rat IgM, hamster IgG, hamster IgM, rabbit IgG, rabbit IgM, goat IgG, goat IgM, sheep IgG, sheep IgM, cattle IgG, cattle IgM, and chicken IgY, which are derived from organisms, such as mice, rats, hamsters, goats, rabbits, sheep, cattle, and chickens, in which an immunoreaction can occur. Either of polyclonal antibodies or monoclonal antibodies can be used. A fragmented antibody is a molecule which has at least one antigen binding portion and is derived from a complete type antibody, and is specifically Fab, F(ab')2, or the like. These complete type antibodies are molecules which are obtained through enzymatic treatment or chemical treatment, or through a genetic engineering technique.

### (Substance Having Binding Properties with respect to First Binding Substance)

The substance having binding properties with respect to the first binding substance is not particularly limited, and preferred examples thereof include an antigen, an antibody, or a complex. The method of preparing an antibody and the type of an antibody are the same as those in the contents disclosed for the third binding substance.

### (Fourth Binding Substance)

The fourth binding substance is a substance which does not have binding properties with respect to a test substance and has binding properties with respect to the first binding substance. As the fourth binding substance, it is preferable to use a compound, for example, an antibody against the first binding substance (antibody) or a protein (Protein A or Protein G) binding to a binding substance (antibody), which has affinity to the first binding substance. Among them, an antibody can be more preferably used. The method of preparing an antibody and the type of an antibody are the same as those in the contents disclosed for the third binding substance. In addition, it is possible to preferably use a compound, in which a part of the first binding substance bound to the first particles having a label, and the fourth binding substance are in a ligand and non-ligand relationship.

### (Method of Immobilizing Binding Substance on Substrate)

The method of immobilizing the third binding substance and the fourth binding substance such as an antibody on a substrate is disclosed in, for example, Tech Notes Vol. 2-12 or the like which is provided by Nunc, and any well-known method of preparing a general Enzyme-Linked ImmunoSorbent Assay (ELISA) reagent can be used. In addition, surface modification through arranging self-assembled monolayers (SAM) or the like on a substrate may be performed. As a method of immobilizing an antibody as a binding substance on a substrate, any method such as a method using physical adsorption or a method using chemical binding through covalent bonding can also be employed. As a blocking agent which covers the surface of a substrate which is not coated with an antibody after immobilizing the antibody on the substrate, well-known substances, for example, bovine serum albumin (BSA), skim milk, casein, soy-derived components, fish-derived components, or polyethylene glycol, or commercially available blocking agents for immunoreaction containing thease substances or substances having the same characteristics as these substances can be used. It is possible to perform pretreatment, such as partial modification through heat, acid/alkali or the like, on these blocking agents as necessary.

### (Substrate)

The substrate used in the present invention is a substrate on which a metal film is formed, and the form thereof is not particularly limited. When performing a fluorescence detection method using surface plasmon excitation (SPF method) to be described later, it is preferable to use a substrate having a flow path to be described later and a metal film which is a reaction site on the surface. As a metal constituting the metal film, it is possible to use a substance in which surface plasmon resonance may occur. Preferred examples of the metal include gold, silver, copper, aluminum, platinum, or the like, and gold is particularly preferable. The above-described metals can be used singly or in combination. In addition, an intermediate layer formed of chromium or the like may be provided between the substrate and a layer formed of metal in consideration of adhesiveness to the above-described substrate. The thickness of the metal film is not particularly limited. For example, the thickness of the metal film is preferably 0.1 nm to 500 nm, more preferably 1 nm to 200 nm, and particularly preferably 1 nm to 100 nm. When the thickness of the metal film exceeds 500 nm, it is impossible to sufficiently detect a surface plasmon phenomenon of a medium. In addition, in a case of providing the intermediate layer formed of chromium or the like, the thickness of the intermediate layer is preferably 0.1 nm to 10 nm.

The formation of the metal film may be performed through a usual method. For example, the formation of the metal film can be performed through a sputtering method, an evaporation method, an ion plating method, an electroplating method, and an electroless plating method. In order to realize good adhesiveness of the metal film to a substrate, it is preferable to form the metal film through a sputtering method.

The metal film is disposed on a substrate. Here, being "disposed on a substrate" includes a case where the metal film is disposed with other layers therebetween without directly coming into contact with the substrate in addition to a case where the metal film is disposed on the substrate so as to come into contact with the substrate. As the substrate which can be used in the present invention, for example, it is possible to use optical glass such as BK7 (borosilicate glass) which is a type of general optical glass, or a synthetic resin, and specifically a substrate formed of a material, such as polymethyl methacrylate, polyethylene terephthalate, polycarbonate, and a cycloolefin polymer, which is transparent to laser light. As such a substrate, a material is preferable which does not exhibit anisotropy with respect to polarized light and is excellent in processability. Examples of the substrate for allowing detection of fluorescence through a SPF method include a substrate or the like which is obtained by producing a gold film on polymethyl methacrylate through a sputtering method.

### [Method of Measuring Test Substance]

The method of measuring a test substance according to the present invention includes: a step of bringing a solution, which contains a test substance, first particles which are modified by a first binding substance having specific binding properties with respect to the test substance and have a label, second particles which are modified by a second binding substance which does not have specific binding properties with respect to the test substance, the second particles not having a label, and a compound which has at least one amino group having a positive charge, and has an isoelectric point greater than 7, into contact with a first metal film to which a third binding substance having specific binding properties with respect to the test substance, or a substance having binding properties with respect to the first binding substance is immobilized; and a step of detecting a signal in accordance with the label from the first metal film, wherein the first binding substance is anti-ACTH antibody, wherein the second binding substance is anti-CRP antibody, wherein the test substance is adrenocorticotropic hormone, and wherein the mass ratio of the second particles to the first particles is 1 to 6.

In addition, the method of measuring a test substance according to the present invention may further include: a step of bringing the above-described solution into contact with a second metal film to which a fourth binding substance which does not have binding properties with respect to the above-described test substance and has binding properties with respect to the first binding substance is immobilized; a step of detecting a signal in accordance with the above-described label from the above-described second metal film; and a step of correcting the signal detected from the above-described first metal film using the signal detected from the above-described second metal film.

### (Measurement Method)

The measurement method of the present invention is interpreted as the widest concept including detecting whether there is a test substance or measuring (that is, quantitatively determining) the amount of a test substance. Examples of a specific embodiment of the measurement method of the present invention include a sandwich method or a competition method, and a sandwich method is preferable.

### <Sandwich Method>

The sandwich method is not particularly limited. For example, it is possible to measure a test substance through the following procedure. First, a first binding substance having specific binding properties with respect to a test substance, and a third binding substance having specific binding properties with respect to the test substance are prepared in advance. The first binding substance binds to first particles having a label to produce first particles which are modified by the first binding substance having specific binding properties with respect to the test substance. Next, the third binding substance is prepared and immobilized on a substrate which is set to be a reaction site (test area). In addition, a fourth binding substance is prepared and immobilized on a substrate which is set to be a control area. A second binding substance which does not have specific binding properties with respect to the test substance is separately prepared and binds to second particles which do not have a label to produce second particles which do not have a label and are modified by the second binding substance which does not have specific binding properties with respect to the test substance. The above-described first particles and the above-described second particles are mixed and stored in a container, and are then dried. A test sample (or extraction liquid thereof) which may contain the test substance, and a mixture of the first particles and the second particles are mixed and dissolution is performed, the mixed dissolution solution is applied to the substrate, and the top of the flow path on the substrate is developed and is brought into contact with the reaction site. In a case where there is a test substance in the test sample, a reaction (antigen-antibody reaction when using an antigen and an antibody) between the test substance and the first binding substance which binds to the first particles and between the test substance and the third binding substance on the reaction site occurs in the reaction site and the amount of the first particles corresponding to the amount of the test substance is immobilized on the reaction site. In the sandwich method, after completion of the reaction between the test substance and the third binding substance which has been immobilized on the reaction site, and the reaction between the test substance and the first binding substance which has been bound to the first particles, it is also possible to perform washing for the purpose of removing first particles which have not been bound in the test area and the control area on the substrate. Next, it is possible to measure the concentration of the test substance accurately by detecting the intensity of a signal from the first particles which have been bound to the reaction site. The intensity of fluorescence and the concentration of the test substance have a positive correlation.

### <Competition Method>

The competition method is not particularly limited. For example, it is possible to measure a test substance through the following procedure. The competition method is well known in the art as a technique of detecting an antigen of a low molecular compound which cannot be assayed by the sandwich method. First, a first binding substance having specific binding properties with respect to a test substance and a second binding substance which does not have specific binding properties with respect to the test substance are prepared in advance. Next, the first binding substance binds to first particles and the second binding substance binds to second particles. In addition, a compound having an epitope with respect to the test substance itself having binding properties with respect to the first binding substance, or with respect to the first binding substance antibody which has a site similar to the test substance or is similar to the test substance, is immobilized on a substrate which is set to be a reaction site. Next, the first particles and the second particles are mixed and stored in a container, and are then dried. A test sample (or extraction liquid thereof) which may contain the test substance, and a mixture of the first particles and the second particles are mixed and dissolution is performed, the mixed dissolution solution is applied to the substrate, and the top of the flow path on the substrate is developed and is brought into contact with the reaction site. In a case where there is no test substance in the test sample, a reaction occurs on the substrate due to the first binding substance, which has been bound to the first particles, and the similar compound having an epitope with respect to the test substance itself having binding properties with respect to a first binding substance which has been immobilized on the reaction site, or with respect to a first binding substance antibody similar to the test substance. In contrast, in a case where there is a test substance, since the test substance binds to the first binding substance, the reaction, between the first binding substance, which has been bound to the first particles, and the compound having an epitope with respect to the test substance itself having binding properties with respect to the first binding substance, or with respect to the first binding substance antibody which has a site similar to the test substance or is similar to the test substance, on the reaction site is inhibited, and immobilization of the first particles having a label, on the reaction site is inhibited. In the competition method, a plurality of samples with known amounts of test substance which have different concentrations of the test substance are prepared in advance. Fluorescent signals from the reaction site are measured at a plurality of different times while bringing the samples and binding substance-labeled fluorescence particles into contact with the top of the reaction site. The temporal change (inclination) in the amount of fluorescence is obtained from the plurality of measurement results of the concentrations of the test substance. The temporal change is plotted on a Y axis and the concentrations of the test substances are plotted on an X axis and a calibration curve for concentrations of the test substances with respect to the temporal change in the amount of fluorescence is acquired using an appropriate fitting method such as a least-squares method. It is possible to quantitatively determine the amount of the test substances contained in the test samples from the results of the temporal change in the amount of fluorescence, using the target test samples, based on the acquired calibration curve in this manner.

### (Flow Path)

In a preferred mode of the present invention, a mixture, in which a test sample (or extraction liquid thereof) which may contain a test substance, first particles having a label, and second particles which are further added are mixed, are dissolved. It is possible to apply the dissolution solution onto a substrate and to develop the dissolution solution in a flow path. The flow path is not particularly limited as long as the flow path is a passage through which the test sample and the first particles having a label (and the second particles) flow down to a reaction site. A preferred mode for the flow path is a structure in which there is a spotting port through which a test sample solution containing the first particles having a label (and the second particles) is spotted, a metal thin film as a reaction site on which a third binding substance is immobilized, and a flow path over the metal thin film, and the test sample can pass through the top of the metal thin film. Preferably, it is possible to provide a suction port on a side opposite to the spotting port, with respect to the metal thin film.

### (Method of Detecting Signal in accordance with Label)

In the present invention, a signal in accordance with a label is detected. As described above, the label preferably emits fluorescence, and in this case, it is possible to detect the signal in accordance with the label by detecting the fluorescence. As the method of detecting fluorescence, for example, it is preferable to detect the intensity of fluorescence using a microplate reader, or a biosensor for performing a fluorescence detection method using surface plasmon excitation (SPF method). In general, the detection of the intensity of fluorescence is completed after a certain time after antigen-antibody reaction, for example, after several minutes to several hours. It is possible to quantitatively determine the concentration of a test substance (ACTH) from a relationship between the intensity of fluorescence and the concentration of the test substance by detecting the degree of formation of an immune complex as the intensity of fluorescence. The mode of measuring fluorescence may be plate reader measurement or flow measurement. Through the SPF method, it is possible to perform the measurement with sensitivity higher than that in the fluorescence detection method (vertical illumination fluorescence method) through vertical illumination excitation.

As the surface plasmon fluorescence (SPF) biosensor, it is possible to use a sensor which is disclosed in, for example, JP2008-249361A and includes an optical waveguide which is formed of a material through which excitation light of a predetermined wavelength is transmitted; a metal film which is formed on a surface of the optical waveguide; a light source which generates a light beam; an optical system in which the above-described optical beam passes through the optical waveguide and is incident at an incidence angle at which surface plasmons are generated with respect to an interface between the above-described optical waveguide and the metal film; and fluorescence detection means which detects fluorescence that is generated through excitation using an evanescent wave which has been enhanced by the above-described surface plasmons.

### (Method of Measuring Amount of Test Substance)

As an example of a method of quantitatively determining a test substance (ACTH) in the SPF method in the present invention, it is possible to quantitatively determine the test substance through the following method. Specifically, samples containing test substances at known concentrations are prepared and fluorescence signals from a site at which fluorescence is detected are measured at a plurality of different times while making the samples flow down to the fluorescence detection site. The temporal change (inclination) in the amount of fluorescence is obtained from a plurality of measurement results of the concentrations of the test substances. The temporal change is plotted on a Y axis and the concentrations of the test substances are plotted on an X axis and a calibration curve for concentrations of the test substances with respect to the temporal change in the amount of fluorescence is acquired using an appropriate fitting method such as a least-squares method. In the light signal system, it is possible to specify the amount of the test substances of the target test samples based on the calibration curve corresponding to the test substance.

The surface plasmon excitation fluorescence detection (SPF) system in the present invention is an assay method of detecting fluorescence from a fluorescent substance which depends on the amount of a test substance (ACTH) immobilized on a metal thin film on a substrate, and is a method which is different from a latex aggregation method, that is, a method of detecting the optical change in transparency as, for example, turbidity, through the progress of a reaction in a solution. In the latex aggregation method, antibody-sensitized latex in a latex reagent and an antigen in a specimen are aggregated by being bound through an antibody reaction. A system, in which an aggregate becomes larger over time and the concentration of the antigen is quantitatively determined through an absorbance change per unit time which is obtained by irradiating the aggregate with near infrared light, is the latex aggregation method. In the present invention, it is possible to provide an extremely simple method of detecting a test substance compared to the latex aggregation method.

The present invention will be more specifically described using the following examples, but the present invention is not limited to the examples.

### [Examples]

### Example 1

### (1) Production of Latex Particles with Average Particle Size of 260 nm

30 g (288 mmol) of styrene (manufactured by Wako Pure Chemical Industries, Ltd.) and 1 g (12 mmol) of acrylic acid (manufactured by Wako Pure Chemical Industries, Ltd.) were suspended in 330 mL of ultrapure water. The temperature of the obtained liquid mixture was increased to 85°C, and 25 mL of an aqueous solution in which 1 g of potassium persulfate (manufactured by Wako Pure Chemical Industries, Ltd.) was dissolved was added thereto. The obtained liquid mixture was stirred for 6 hours at 250 rpm (rotations/min) at 85°C. Thereafter, the stirred liquid mixture was subjected to centrifugal separation under the conditions of 25°C, 10,000 rpm, and 6 hours, a supernatant liquid was removed therefrom, 10 mL of ultrapure water was added to the remaining precipitate, and the liquid mixture was rinsed by agitation. The process from the centrifugal separation to the rinsing was repeated three times in total. The liquid mixture after the rinsing was subjected to centrifugal separation under the conditions of 25°C, 10,000 rpm, and 6 hours, a supernatant liquid was removed therefrom, and the precipitate was dispersed again in ultrapure water to obtain latex particles. A dilute solution was prepared by adding ultrapure water thereto such that the solid content concentration of the obtained latex particles became 1 mass%. The median diameter (50% diameter, d50) was obtained at a temperature of 25°C using particle size analyzer FPAR-1000 (Otsuka Electronics Co., Ltd.). The result of the measurement was that the average particle size of the latex particles was 260 nm.

### (2) Production of Latex Particles with Average Particle Size of 150 nm

30 g (288 mmol) of styrene (manufactured by Wako Pure Chemical Industries, Ltd.) and 3 g (42 mmol) of an acrylic acid (manufactured by Wako Pure Chemical Industries, Ltd.) were suspended in 440 mL of ultrapure water. The temperature of the obtained liquid mixture was increased to 95°C, and 10 mL of an aqueous solution in which 1 g of potassium persulfate (manufactured by Wako Pure Chemical Industries, Ltd.) was dissolved was added thereto. The obtained liquid mixture was stirred for 6 hours at 250 rpm at 95°C. Thereafter, the stirred liquid mixture was subjected to centrifugal separation under the conditions of 25°C, 10,000 rpm, and 6 hours, a supernatant liquid was removed therefrom, 10 mL of ultrapure water was added to the remaining precipitate, and the liquid mixture was rinsed by agitation. The process from the centrifugal separation to the rinsing was repeated three times in total. The liquid mixture after the rinsing was subjected to centrifugal separation under the conditions of 25°C, 10,000 rpm, and 6 hours, a supernatant liquid was removed therefrom, and the precipitate was dispersed again in ultrapure water to obtain latex particles. A dilute solution was prepared by adding ultrapure water thereto such that the solid content concentration of the obtained latex particles became 1 mass%. The median diameter (50% diameter, d50) was obtained at a temperature of 25°C using particle size analyzer FPAR-1000 (Otsuka Electronics Co., Ltd.). The result of the measurement was that the average particle size of the latex particles was 150 nm.

### (3) Production of Fluorescent Latex Particles

100 mL of an aqueous dispersion, in which the solid content concentration of the latex particles with an average particle size of 260 nm which was produced in the above-described (1) was adjusted using ultrapure water so as to become 2 mass%, was prepared. 100 mL of methanol was gradually added thereto by a constant amount at constant intervals for 10 minutes at 25°C while being stirred, to obtain a latex dispersion. A fluorescent pigment (NK136, manufactured by Hayashibara Seibutsu Kagaku Kenkyujo) solution (which was obtained by dissolving 10 mg of NK136 in 1 mL of N,N-dimethylformamide, 9 mL of CHCl₃, and 16 mL of ethanol) which was separately prepared was gradually dripped into the latex dispersion in constant amounts at constant intervals for 60 minutes to obtain a liquid mixture of the latex dispersion and the fluorescent pigment. Next, an organic solvent was distilled under reduced pressure using an evaporator with respect to the liquid mixture of the latex dispersion and the fluorescent pigment. Thereafter, the liquid mixture of the latex dispersion and the fluorescent pigment from which the organic solvent was distilled under reduced pressure was subjected to centrifugal separation under the conditions of 25°C, 10,000 rpm, and 6 hours and a supernatant liquid was removed therefrom. Then, the precipitate was dispersed again in phosphate buffered saline (PBS solution). This operation was repeated three times in total, and production of 2 mass% fluorescent latex particles was completed.

### (4) Production of Fluorescent Latex Particles Which are Modified by Antibody (Anti-ACTH Antibody) with respect to Adrenocorticotropic Hormone

88 µL of a 50 mmol/L 2-morpholioethanesulfonic acid (MES) buffer (pH 6.6) solution was added to 275 µL of a 2 mass% (solid content concentration) fluorescent latex particle aqueous solution (average particle size of 260 nm). 176 µL of a 5 mg/mL anti-ACTH monoclonal antibody (anti-ACTH MC 49039-7; the production method will be described later) was added to the obtained liquid mixture. The obtained liquid mixture was stirred for 15 minutes at room temperature. Thereafter, 5 µL of a 10 mg/mL EDC (1-ethyl-3-(3-dimethylaminopropyl)carbodiimide hydrochloride; manufactured by Wako Pure Chemical Industries, Ltd.) aqueous solution was added thereto and the obtained liquid mixture was stirred for 2 hours at room temperature. 8.8 µL of a 2 mol/L glycine (manufactured by Wako Pure Chemical Industries, Ltd.) aqueous solution was added thereto and the obtained liquid mixture was stirred for 15 minutes. Then, the stirred liquid mixture was subjected to centrifugal separation (15,000 rpm, 4°C, 15 minutes) to precipitate the fluorescent latex particles. Thereafter, a supernatant liquid was removed, 500 µL of a PBS solution (pH 7.4) was added to the precipitate, and the fluorescent latex particles were dispersed again using an ultrasonic cleaning machine. After removing a supernatant liquid again by performing the centrifugal separation (15,000 rpm, 4°C, 15 minutes), 500 µL of a PBS (pH 7.4) solution containing 1 mass% bovine serum albumin (BSA) was added to the precipitate and the fluorescent latex particles were dispersed again. 1 mass% anti-ACTH antibody binding fluorescent latex particle solution was prepared in this manner.

### (Production of Monoclonal Antibody)

The anti-ACTH monoclonal antibody (anti-ACTH MC 49039-7) and an anti-CRP (C-reactive protein) monoclonal antibody (MM50175) to be described later were produced as follows using a mouse ascites method.

ACTH was chemically synthesized, a mouse was immunized with the chemically synthesized ACTH. Then, a spleen cell was extracted from the mouse and was mixed with myeloma (product name: P3-X63-Ag8-U1, manufactured by Cosmo Bio Co., Ltd.), and cell fusion was performed through a polyethylene glycol (PEG) method. The cell used here was a spleen cell collected 3 days after final immunization. The cell ratio was set to spleen cell:myeloma = 10:1. Cell seeding was performed using spleen cells in a 96-well plate at 0.5 to 1.0 x 10⁵ cells/well. As a medium used, RPMI-1640 (Roswell Park Memorial Institute medium), 10% fetal bovine serum (FBS), and HAT (hypoxanthine-aminopterin-thymidine-mixed medium) were appropriately selected. Finally, the proliferated fused cell (hybridoma) was injected into the abdominal cavity of the mouse, abdominal dropsy was taken out after the elapse of a constant period of time, and then, an antibody which was obtained through centrifugal separation and purification processes was collected as the anti-ACTH monoclonal antibody. In addition, similarly to the production of the anti-ACTH monoclonal antibody, an anti-CRP monoclonal antibody was produced using CPR (manufactured by Oriental Yeast Co., Ltd.) which was purified from a culture solution of Escherichia coli.

### (5) Preparation of Latex Particles Which are Modified by Anti-CRP Antibody Which is not Fluorescently Labeled

96 µL of a 250 mmol/L MES buffer (pH 5.6) solution and 25 mL of ultrapure water were added to 300 µL of a 2 mass% (solid content concentration) latex particle aqueous solution (average particle size of 150 nm). 182 µL of a 5 mg/mL anti-CRP monoclonal antibody (MM 50175) was added to the obtained liquid mixture. Next, the obtained liquid mixture was stirred for 15 minutes at room temperature. Thereafter, 9.6 µL of a 10 mg/mL EDC aqueous solution was added thereto and the obtained liquid mixture was stirred for 2 hours at room temperature. 30 µL of a 2 mol/L glycine (manufactured by Wako Pure Chemical Industries, Ltd.) aqueous solution was added thereto and the obtained liquid mixture was stirred for 30 minutes. Then, the stirred liquid mixture was subjected to centrifugal separation (15,000 rpm, 4°C, 15 minutes) to precipitate the latex particles. A supernatant liquid was removed, 600 µL of a PBS solution (pH 7.4) was added to the precipitate, and the latex particles were dispersed again using an ultrasonic cleaning machine. After removing a supernatant liquid again by performing the centrifugal separation (15,000 rpm, 4°C, 15 minutes), 600 µL of a PBS (pH 7.4) solution containing 1 mass% BSA was added to the precipitate and the latex particles were dispersed again. 1 mass% anti-CRP antibody binding latex particle solution was prepared in this manner.

### (6) Preparation of Dried Particles from Fluorescently Labeled Particles and Fluorescent Label-free Particles

240 µL of ultrapure water, 417 µL of a 20 mass% sucrose aqueous solution, 229 µL of a 20 mass% BSA aqueous solution, 125 µL of a 1 mass% anti-ACTH antibody-modified fluorescent latex particle (average particle size of 260 nm) solution, and 554 µL of an 1 mass% anti-CRP antibody-modified latex particles (average particle size of 150 nm) were mixed to prepare a liquid mixture A. A cup of which a base body was set to be formed of polypropylene (manufactured by Prime Polymer Co., Ltd., Prime Polypro (registered trade name); random PP grade) was prepared and 20 µL of the above-described liquid mixture A was added to the cup. Thereafter, the liquid mixture was dried for 12 hours using a super dry dryer (manufactured by Toyo living; Ultra Super Dry 00 Series) to produce dried particles, of fluorescently labeled particles and fluorescent label-free particles, of which the water content was set to less than or equal to 25 mass%.

### (7) Production of Substrate

A gold film which is used for a test area and a gold film which is used for a control area and is formed next to the gold film used for the test area were produced on a single surface of a substrate, of which a base body was set to be formed of polymethyl methacrylate (manufactured by Mitsubishi Rayon Co., Ltd.; Acrypet (registered trade name) VH-001), through a magnetron sputtering method such that both the test area and the control area had a width of 4 mm and a thickness of 36 nm. The substrate was cut and produced so as to have a width of 5 mm. A solution (concentration: 10 µg/mL in 150 mmol/L of NaCl) containing an anti-ACTH monoclonal antibody (manufactured by Yamasa Corporation; 7595) as a third binding substance was spotted on the gold film of the test area on the substrate, and an antibody was physically adsorbed on the gold film under the conditions of 1 hour and 25°C. Similarly, a solution (concentration: 10 µg/mL in 150 mmol/L of NaCI) containing an anti-mouse antibody (anti-mouse IgG F(ab')2, product name: AffiniPure F(ab')2 Fragment Rabbit Anti-mouse IgG (H+L), manufactured by Jackson ImmunoResearch Inc.) as a fourth binding substance was spotted on the gold film of the control area, and an antibody was physically adsorbed on the metal film under the conditions of 1 hour and 25°C.

### (8) Washing and Blocking of Substrate

The substrate produced in (7) was washed using 300 µL of a PBS solution (pH 7.4) containing a washing solution (0.05 mass% Tween (registered trade name) 20 (polyoxyethylene (20) sorbitan monolaurate, manufactured by Wako Pure Chemical Industries, Ltd.) which has been prepared in advance. The washing was repeatedly performed three times. After the completion of the washing, 300 µL of a PBS solution (pH 7.4) containing 1 mass% casein (manufactured by Thermo Scientific Inc.) was added to the substrate which was then allowed to stand under the conditions of 1 hour and 25°C, in order to perform blocking of a portion on the gold films in which the antibodies are not adsorbed. Thereafter, the substrate was washed again using a PBS solution (pH 7.4) containing a washing solution (0.05 mass% Tween 20 (polyoxyethylene (20) sorbitan monolaurate, manufactured by Wako Pure Chemical Industries, Ltd.). Then, 300 µL of Immunoassay Stabilizer (manufactured by ABI Inc.) was added to the substrate as a stabilizer, and the substrate was then allowed to stand for 30 minutes under the condition of 25°C. Thereafter, moisture was completely removed from the substrate using a dryer after removing the solution.

### (9) Production of Sensor Chip

The produced substrate was sealed in a flow path and a flow path-type sensor chip was produced so as to have a configuration in a second embodiment of JP2010-190880A.

### (10) Preparation of Test Samples

A test sample 1 and a test sample 2 were prepared which have different concentrations of test substances, using oriental beagle plasma which was purchased from Kitayama Labes Co., Ltd as dog plasma.

### (11) Immunoassay of ACTH using Fluorescent Particles

100 µL of each of the test samples 1 and 2 (dog plasma) prepared in (10) and 25 µmol (5.27 mg) of L-(+) arginine hydrochloride (manufactured by Wako Pure Chemical Industries, Ltd.; molecular weight of 210.66) were sufficiently mixed to produce mixed samples such that the concentration of L-(+) arginine hydrochloride became 250 mmol/L. Next, cups into which the anti-ACTH antibody-labeled fluorescent-dried particles and the anti-CRP antibody label-dried particles produced in (6) were respectively put were stored for 15 days in an environment of 50% relative humidity at 25°C. Then, the mixed samples produced in the above were added to the cups and the liquid mixtures were stirred for 10 minutes. The obtained liquid mixtures were spotted on the flow path-type sensor chip produced in (9). After the spotting, the liquid mixtures were made to flow down at a rate of 10 µL/minute while performing pump suctioning. The amount (signal [mA/minute]) of change in the intensity of fluorescence per hour on the surface of each gold film on which an ACTH antibody is immobilized was continuously measured for 1.5 minutes while making the liquid mixtures flow down. A signal which was obtained from the test area of each substrate was corrected using a signal of the control area. A corrected value of the increase rate in a unit time was obtained as a corrected result. Creation of a calibration curve and calculation of the concentration of the ACTH in each test sample were performed using fluorescent signal-corrected values.

### (12) Measurement using Contrasting Device

Immulyze 1000 which is a totally automatic immunochemiluminescent measurement device manufactured by Siemens Japan K.K. and had been widely used by those skilled in the art was used as a contrasting device, and the concentrations of the test substances in the test samples 1 and 2 which were prepared in the above (10) were measured in accordance with an instruction manual. The results are shown in Table 1.

**[Table 1]**

| | Concentration of ACTH (pg/mL) |
|---|---|
| Test sample 1 | <10 (regarded as 0) |
| Test sample 2 | 300 |

### (13) Evaluation of Sensitivity

From the measurement values measured by the contrasting device, the concentration of ACTH of test sample 1 was low, and therefore, it was possible to regard the concentration thereof as zero. In contrast, it was found that the concentration of ACTH of test sample 2 was comparatively high. Fluorescent signal values which were obtained from the test areas of the test samples 1 and 2 obtained in the immunoassay of ACTH using the fluorescent particles in (11) were respectively represented by N (noise) and S (signal), and the obtained ratio (S/N) was evaluated based on the following criteria. The results are shown in Table 2. It can be seen that, regarding the measurement sensitivity, it is possible to perform higher sensitivity measurement as the ratio of the value of S, in which the concentration of ACTH is comparatively high, with respect to the value of N, in which the concentration of ACTH is low (about zero), becomes larger.

Evaluation Criteria of sensitivity:
C: When S/N is less than 5
B: When S/N is greater than or equal to 5 and less than 8
A: When S/N is greater than or equal to 8

### Examples 2 to 4 and Comparative Examples 1 to 3

Examples 2 to 4 and Comparative Examples 1 to 3 were carried out similarly to Example 1 except that additive substances shown in Table 2 were added to each test sample instead of arginine hydrochloride in Example 1 such that each molar concentration became the same as that of arginine hydrochloride in Example 1. The results of the evaluation of the sensitivity are shown in Table 2.

**[Table 2]**

| | Additive substance | Signal [mA/minute] | | S/N | Evaluation |
|---|---|---|---|---|---|
| | | Test sample 1 (N) | Test sample 2 (S) | | |
| Example 1 | Arginine | 39.9 | 452.6 | 11.3 | A |
| Example 2 | Lysine | 46.7 | 486.1 | 10.4 | A |
| Example 3 | Guanidine hydrochloride | 76.0 | 570.9 | 7.5 | B |
| Example 4 | Ornithine | 105.0 | 766.4 | 7.3 | B |
| Comparative Example 1 | Sodium chloride | 301.0 | 951.4 | 3.2 | C |
| Comparative Example 2 | Glycine | 200.2 | 839.7 | 4.2 | C |
| Comparative Example 3 | Magnesium chloride | 22.1 | 28.3 | 1.3 | C |

From the results of Table 2, it was confirmed that there is an effect of improving the measurement sensitivity by the compound which has at least one amino group having a positive charge of the present invention. Particularly, it is obvious from the results of Table 2 that arginine and lysine exhibit an excellent effect. With magnesium chloride, the reactivity between ACTH and an antibody is remarkably decreased while it is possible to suppress increase in segments. It can also be seen that there is no effect obtained by glycine.

### Examples 5 to 11 and Comparative Example 4

Examples 5 to 11 and Comparative Example 4 (without adding arginine) were carried out similarly to Example 1 except that the added concentration of arginine in Example 1 was changed from 250 mmol/L. The results of the evaluation of the sensitivity are shown in Table 3 (Example 1 is also described as well).

**[Table 3]**

| | Additive substance | Additive concentration | Signal [mA/minute] | | S/N | Evaluation |
|---|---|---|---|---|---|---|
| | | | Test sample 1 (N) | Test sample 2 (S) | | |
| Comparative Example 4 | - | - | 2553.9 | 1898.2 | 0.7 | C |
| Example 5 | Arginine | 100 | 127.4 | 954.6 | 7.5 | B |
| Example 6 | Arginine | 150 | 50.8 | 859.4 | 16.9 | A |
| Example 7 | Arginine | 200 | 47.2 | 583.7 | 12.4 | A |
| Example 1 | Arginine | 250 | 39.9 | 452.6 | 11.3 | A |
| Example 8 | Arginine | 275 | 36.6 | 302.0 | 8.3 | A |
| Example 9 | Arginine | 300 | 43.6 | 283.0 | 6.5 | B |
| Example 10 | Arginine | 350 | 37.7 | 241.3 | 6.4 | B |
| Example 11 | Arginine | 400 | 34.1 | 194.7 | 5.7 | B |

From the results of Table 3, it can be seen that S (signal of test sample 2) decreases further while N (signal of test sample 1) improves further as the amount of arginine added becomes larger, and therefore, S/N improves to an optimal value as the added concentration becomes higher.

## Claims

1. A reagent kit which is used for measuring a test substance in a biological sample, comprising:
first particles which are modified by a first binding substance having specific binding properties with respect to the test substance and have a label;
second particles which are modified by a second binding substance which does not have specific binding properties with respect to the test substance, the second particles not having a label, and
a compound which has at least one amino group having a positive charge, and has an isoelectric point greater than 7,
wherein the first binding substance is an anti-ACTH antibody,
wherein the second binding substance is an anti-CRP antibody,
wherein the test substance is adrenocorticotropic hormone, and
wherein the mass ratio of the second particles to the first particles is 1 to 6.

2. The reagent kit according to claim 1,
wherein the compound is an amino acid.

3. The reagent kit according to claim 2,
wherein the amino acid is at least one compound which is selected from arginine, histidine, lysine, and ornithine.

4. The reagent kit according to any one of claims 1 to 3,
wherein the particle size of the first particles is 200 nm to 300 nm.

5. The reagent kit according to any one of claims 1 to 4,
wherein the label contains a fluorescent pigment.

6. The reagent kit according to any one of claims 1 to 5,
wherein the particle size of the second particles is 100 nm to 200 nm.

7. The reagent kit according to any one of claims 1 to 6,
wherein the amount of the compound, which has at least one amino group having a positive charge and has an isoelectric point greater than 7, used is 100 mmol/L to 400 mmol/L in terms of concentration in a reaction liquid.

8. A measurement kit which is used for measuring a test substance in a biological sample, comprising:
the reagent kit according to any one of claims 1 to 7; and
a substrate on which a first metal film, to which a third binding substance having specific binding properties with respect to the test substance, or a substance having binding properties with respect to the first binding substance, is immobilized is formed.

9. The measurement kit according to claim 8,
wherein a second metal film, to which a fourth binding substance which does not have binding properties with respect to the test substance and has binding properties with respect to the first binding substance, is immobilized is further formed on the substrate.

10. A method of measuring a test substance, comprising:
a step of bringing a solution, which contains a test substance, first particles which are modified by a first binding substance having specific binding properties with respect to the test substance and have a label, second particles which are modified by a second binding substance which does not have specific binding properties with respect to the test substance, the second particles not having a label, and a compound which has at least one amino group having a positive charge, and has an isoelectric point greater than 7, into contact with a first metal film to which a third binding substance having specific binding properties with respect to the test substance, or a substance having binding properties with respect to the first binding substance is immobilized; and
a step of detecting a signal in accordance with the label from the first metal film,
wherein the first binding substance is an anti-ACTH antibody,
wherein the second binding substance is an anti-CRP antibody,
wherein the test substance is adrenocorticotropic hormone, and
wherein the mass ratio of the second particles to the first particles is 1 to 6.

11. The measurement method according to claim 10, further comprising:
a step of bringing the solution into contact with a second metal film to which a fourth binding substance which does not have binding properties with respect to the test substance and has binding properties with respect to the first binding substance is immobilized;
a step of detecting a signal in accordance with the label from the second metal film; and
a step of correcting the signal detected from the first metal film using the signal detected from the second metal film.

## Patentansprüche

1. Reagenzkit, das zur Bestimmung einer Testsubstanz in einer biologischen Probe verwendet wird, aufweisend:
erste Partikel, die modifiziert sind mittels einer ersten Bindungssubstanz mit spezifischen Bindungseigenschaften bezüglich der Testsubstanz, und die eine Markierung haben;
zweite Partikel, die modifiziert sind mittels einer zweiten Bindungssubstanz ohne spezifische Bindungseigenschaften bezüglich der Testsubstanz, wobei die zweiten Partikel keine Markierung haben, und
eine Verbindung, die mindestens eine Aminogruppe mit einer positiven Ladung hat, und die einen isoelektrischen Punkt von größer als 7 hat,
wobei die erste Bindungssubstanz ein Anti-ACTH-Antikörper ist,
wobei die zweite Bindungssubstanz ein Anti-CRP-Antikörper ist,
wobei die Testsubstanz adrenocorticotropes Hormon ist, und
wobei das Massenverhältnis der zweiten Partikel zu den ersten Partikeln 1 bis 6 ist.

2. Reagenzkit nach Anspruch 1,
wobei die Verbindung eine Aminosäure ist.

3. Reagenzkit nach Anspruch 2,
wobei die Aminosäure mindestens eine Verbindung ist, die ausgewählt ist aus Arginin, Histidin, Lysin und Ornithin.

4. Reagenzkit nach einem der Ansprüche 1 bis 3,
wobei die Partikelgröße der ersten Partikel 200 nm bis 300 nm beträgt.

5. Reagenzkit nach einem der Ansprüche 1 bis 4,
wobei die Markierung ein Fluoreszenzpigment enthält.

6. Reagenzkit nach einem der Ansprüche 1 bis 5,
wobei die Partikelgröße der zweiten Partikel 100 nm bis 200 nm beträgt.

7. Reagenzkit nach einem der Ansprüche 1 bis 6,
wobei die Menge der Verbindung, die mindestens eine Aminogruppe mit einer positiven Ladung hat und einen isoelektrischen Punkt von größer als 7 hat, die verwendet wird, 100 mmol/L bis 400 mmol/L beträgt, ausgedrückt als Konzentration in einer Reaktionsflüssigkeit.

8. Bestimmungskit, das verwendet wird zur Bestimmung einer Testsubstanz in einer biologischen Probe, aufweisend:
das Reagenzkit nach einem der Ansprüche 1 bis 7; und
ein Substrat, auf dem ein erster Metallfilm ausgebildet ist, an dem eine dritte Bindungssubstanz mit spezifischen Bindungseigenschaften bezüglich der Testsubstanz, oder eine Substanz mit Bindungseigenschaften bezüglich der ersten Bindungssubstanz immobilisiert ist.

9. Bestimmungskit nach Anspruch 8,
wobei auf dem Substrat außerdem ein zweiter Metallfilm ausgebildet ist, an dem eine vierte Bindungssubstanz ohne Bindungseigenschaften bezüglich der Testsubstanz und mit Bindungseigenschaften bezüglich der ersten Bindungssubstanz immobilisiert ist.

10. Verfahren zur Bestimmung einer Testsubstanz, aufweisend:
einen Schritt des Bringens einer Lösung, die eine Testsubstanz, erste Partikel, die modifiziert sind mittels einer ersten Bindungssubstanz mit spezifischen Bindungseigenschaften bezüglich der Testsubstanz, und die eine Markierung haben, zweite Partikel, die modifiziert sind mittels einer zweiten Bindungssubstanz ohne spezifische Bindungseigenschaften bezüglich der Testsubstanz, wobei die zweiten Partikel keine Markierung haben, und eine Verbindung, die mindestens eine Aminogruppe mit einer positiven Ladung hat und einen isoelektrischen Punkt von größer als 7 hat, enthält, in Kontakt mit einem ersten Metallfilm, an dem eine dritte Bindungssubstanz mit spezifischen Bindungseigenschaften bezüglich der Testsubstanz, oder eine Substanz mit Bindungseigenschaften bezüglich der ersten Bindungssubstanz, immobilisiert ist; und
einen Schritt des Detektierens eines der Markierung entsprechenden Signals, das von dem ersten Metallfilm kommt,
wobei die erste Bindungssubstanz ein Anti-ACTH-Antikörper ist,
wobei die zweite Bindungssubstanz ein Anti-CRP-Antikörper ist,
wobei die Testsubstanz adrenocorticotropes Hormon ist, und
wobei das Massenverhältnis der zweiten Partikel zu den ersten Partikeln 1 bis 6 beträgt.

11. Bestimmungsverfahren nach Anspruch 10, außerdem aufweisend:
einen Schritt des Bringens der Lösung in Kontakt mit einem zweiten Metallfilm, an dem eine vierte Bindungssubstanz ohne Bindungseigenschaften bezüglich der Testsubstanz, und mit Bindungseigenschaften bezüglich der ersten Bindungssubstanz, immobilisiert ist;
einen Schritt des Detektierens eines der Markierung entsprechenden Signals, das von dem zweiten Metallfilm kommt; und
einen Schritt des Korrigierens des von dem ersten Metallfilm detektierten Signals unter Verwendung des von dem zweiten Metallfilm detektierten Signals.

## Revendications

1. Kit de réactif, lequel est utilisé pour mesurer une substance de test dans un échantillon biologique, comprenant :
des premières particules, lesquelles sont modifiées par une première substance liante présentant des propriétés liantes spécifiques par rapport à la substance de test et présentent un marqueur ;
des secondes particules, lesquelles sont modifiées par une deuxième substance liante ne présentant pas de propriétés liantes spécifiques par rapport à la substance de test, les secondes particules ne présentant pas de marqueur ;
un composé, lequel présente au moins un groupe amino présentant une charge positive, et présente un point isoélectrique supérieur à 7 ;
dans lequel la première substance liante est un anticorps anti-ACTH (hormone adrénocorticotrophe) ;
dans lequel la deuxième substance liante est un anticorps anti-CRP ;
dans lequel la substance de test est l'hormone adrénocorticotrophe, et
dans lequel le rapport de masse entre les secondes particules et les premières particules s'étend de 1 à 6.

2. Kit de réactif selon la revendication 1,
dans lequel le composé est un acide aminé.

3. Kit de réactif selon la revendication 2,
dans lequel l'acide aminé est au moins un composé, lequel est sélectionné parmi l'arginine, l'histidine, la lysine, et l'ornithine.

4. Kit de réactif selon l'une quelconque des revendications 1 à 3,
dans lequel la taille de particules des premières particules s'étend de 200 nm à 300 nm.

5. Kit de réactif selon l'une quelconque des revendications 1 à 4,
dans lequel le marqueur contient un pigment fluorescent.

6. Kit de réactif selon l'une quelconque des revendications 1 à 5,
dans lequel la taille de particules des secondes particules s'étend de 100 nm à 200 nm.

7. Kit de réactif selon l'une quelconque des revendications 1 à 6,
dans lequel la quantité du composé utilisé, lequel présente au moins un groupe amino présentant une charge positive et présente un point isoélectrique supérieur à 7, s'étend de 100 mmol/L à 400 mmol/L, en termes de concentration dans un liquide de réaction.

8. Kit de mesure, lequel est utilisé pour mesurer une substance de test dans un échantillon biologique, comprenant :
le kit de réactif selon l'une quelconque des revendications 1 à 7, et
un substrat sur lequel est formé un premier film métallique, sur lequel est immobilisée une troisième substance liante présentant des propriétés liantes spécifiques par rapport à la substance de test, ou une substance présentant des propriétés liantes par rapport à la première substance liante.

9. Kit de mesure selon la revendication 8,
dans lequel un second film métallique, sur lequel est immobilisée une quatrième substance liante ne présentant pas des propriétés liantes spécifiques par rapport à la substance de test, et présentant des propriétés liantes par rapport à la première substance liante, est en outre formé sur le substrat.

10. Procédé de mesure d'une substance de test, comprenant les étapes suivantes :
une étape pour amener une solution, laquelle contient une substance de test, des premières particules, lesquelles sont modifiées par une première substance liante présentant des propriétés liantes spécifiques par rapport à la substance de test et présentent un marqueur, des secondes particules, lesquelles sont modifiées par une deuxième substance liante ne présentant pas de propriétés liantes spécifiques par rapport à la substance de test, les secondes particules ne présentant pas de marqueur, et un composé, lequel présente au moins un groupe amino présentant une charge positive et présente un point isoélectrique supérieur à 7, en contact avec un premier film métallique, sur lequel est immobilisée une troisième substance liante présentant des propriétés liantes spécifiques par rapport à la substance de test, ou une substance présentant des propriétés liantes par rapport à la première substance liante, et
une étape pour détecter un signal conformément au marqueur à partir du premier film métallique ;
dans lequel la première substance liante est un anticorps anti-ACTH ;
dans lequel la deuxième substance liante est un anticorps anti-CRP ;
dans lequel la substance de test est l'hormone adrénocorticotrophe, et
dans lequel le rapport de masse entre les secondes particules et les premières particules s'étend de 1 à 6.

11. Procédé de mesure selon la revendication 10, comprenant en outre :
une étape pour amener une solution en contact avec un second film métallique, sur lequel est immobilisée une quatrième substance liante ne présentant pas de propriétés liantes par rapport à la substance de test, et présentant des propriétés liantes par rapport à la première substance liante ;
une étape pour détecter un signal conformément au marqueur à partir du second film métallique, et
une étape pour corriger le signal détecté à partir du premier film métallique à l'aide du signal détecté à partir du second film métallique.
